# EUROPEAN PATENT APPLICATION

(11) **EP 2 179 736 A1**
(43) Date of publication of application: **28.04.2010**
(21) Application number: 08792254.8
(22) Date of filing: 06.08.2008
(51) Int. Cl.: A61K 31/5383, A61K 9/08, A61P 27/02

(54) **TWO-COMPONENT EYE DROPS CONTAINING PIRENOXINE**

(30) Priority: 09.08.2007 JP 2007208655
(71) Applicant: Senju Pharmaceutical Co., Ltd., Osaka-shi, Osaka 541-0046 (JP)
(72) Inventor: NAKAJIMA, Tomoko, Kobe-shi Hyogo 651-2241 (JP)
(74) Representative: von Kreisler Selting Werner
(86) International application number: PCT/JP2008/064098
(87) International publication number: WO 2009/020145

(57) **Abstract**

A mixed-before-use-type, two-liquid ophthalmic preparation is disclosed consisting of a pirenoxine-containing suspension and a solvent. As compared with those conventional types of ophthalmic preparations with which a tablet or granules containing pirenoxine are dissolved in a solvent before use, the present ophthalmic preparation is **characterized in that** it reduces the time required to dissolve pirenoxine, as well as in that it can give a clear eyedrops with weakly acidic to practically neutral pH, a pH which is physiologically acceptable, after dissolution of pirenoxine.

## Description

### TECHNICAL FIELD

The present invention relates to mixed-before-use-type, two-liquid ophthalmic preparation consisting of a pirenoxine-containing suspension and a solvent.

### BACKGROUND ART

Pirenoxine has been used in the form of ophthalmic preparations for treating senile cataract. As pirenoxine is poorly stable in the form of a solution, such types of its ophthalmic preparations have been provided that are converted to an eyedrops before use by dissolving, in a solvent, a tablet or granules comprising pirenoxine, or in the form of a suspension, so as to maintain its stability through the whole process from their production to distribution and storage, and finally to opening of the their packages by patients. Intending to stabilize pirenoxine, reports have been made of a suspension-type pirenoxine ophthalmic preparation having pH at 3-5.5 and the center of pirenoxine particle size distribution at 0.1-75 µm (Patent document 1) and a suspension ophthalmic preparation which has the center of pirenoxine particle size distribution at 5-100 nm, with the 90%-median size in the particle size distribution being not more than 220 nm, and has pH at 3-6 (Patent document 2).

In the case of the type of pirenoxine ophthalmic preparations with which eyedrops are prepared before use by dissolving, in a solvent, a tablet or granules comprising pirenoxine, it takes time to dissolve pirenoxine. With regard to the suspension-type pirenoxine ophthalmic preparations, not only it is required that their containers be kept upright during storage, but also they must be shaken before use.
[Patent document 1] Japanese Patent Publication No. H07-37386
[Patent document 2] Japanese Patent Application Publication No. 2007-77061

### DISCLOSURE OF THE INVENTION

### [Problem to be solved by the invention]

It is an object of the present invention to provide an ophthalmic preparation with which shorter time is required to dissolve pirenoxine than with those conventional ophthalmic preparations of the type with which solution is prepared before use by dissolving, in a solvent, a tablet or granules comprising pirenoxine.
It is another object of the present invention to provide a pirenoxine-containing ophthalmic preparation which, after dissolution of pirenoxine, gives a clear eyedrops (solution) with weakly acidic to practically neutral pH, which is physiologically acceptable.

### [Means to solve the problem]

As a result of studies made to solve the abovementioned problems, the present inventor found that a mixed-before-use-type, two-liquid ophthalmic preparation consisting of pirenoxine-containing suspension and a solvent enables to reduce the time required to dissolve pirenoxine, as compared with those types of conventional ophthalmic preparations with which a tablet or granules comprising pirenoxine are dissolved in a solvent before use, and, further, can make an ophthalmic preparation which gives a clear eyedrops with weakly acidic to practically neutral pH, which is physiologically acceptable, after dissolution of pirenoxine. The present invention was completed through further studies on the basis of these findings.

Thus, the present invention provides what follows.
(1) A mixed-before-use-type, two-liquid ophthalmic preparation consisting of a pirenoxine-containing suspension and a solvent.
(2) The ophthalmic preparation according to (1) above which is adjusted so that the pH of the clear eyedrops prepared by mixing the pirenoxine-containing suspension and the solvent is 4.5-6.5.
(3) The ophthalmic preparation according to (1) or (2) above, wherein the mixing ratio in volume of the pirenoxine-containing suspension to the solvent is 1:9 to 9:1 (v/v).
(4) The ophthalmic preparation according to one of (1) to (3) above, wherein the concentration of pirenoxine in the pirenoxine-containing suspension is 0.005-0.05 w/v%.
(5) The ophthalmic preparation according to one of (1) to (4) above, wherein the pH of the pirenoxine-containing suspension is 2-4.5.
(6) The ophthalmic preparation according to one of (1) to (5) above, wherein the pH of the solvent is 5-8.
(7) A mixed-before-use-type, two-liquid ophthalmic preparation which is an ophthalmic preparation consisting of a pirenoxine-containing suspension and a solvent, wherein the pirenoxine-containing suspension and the solvent are separately contained in different compartments of a container which is provided with two or more compartments which can be made into communication with one another to mix their contents before use

### [Effect of the invention]

By providing a mixed-before-use-type, two-liquid ophthalmic preparation consisting of a pirenoxine-containing suspension and a solvent, the present invention reduces the time required to dissolve pirenoxine during the dissolution process before use, as compared with conventional ophthalmic preparations of the type with which a tablet or granules comprising pirenoxine are dissolved in a solvent before use.

### BEST MODE FOR CARRYING OUT THE INVENTION

The content of pirenoxine in the pirenoxine-containing suspension according to the present invention is from about 0.005 to about 0.05 w/v%. The center of the pirenoxine particle size distribution in the pirenoxine-containing suspension according to the present invention is 0.005-75 µm, and preferably 0.1-20 µm.

The mixing ratio of the pirenoxine-containing suspension to the solvent according to the present invention is preferably 1:9 to 9:1 in volume (v/v). By way of example, when mixing the pirenoxine-containing suspension with the solvent at a mixing ratio in volume of 1:9 (v/v), one volume of a suspension containing 0.05 w/v% pirenoxine may be mixed with 9 volumes of the solvent.

The pH of the pirenoxine-containing suspension and of the solvent according to the present invention is adjusted with one or more pH adjusting agents so that the pH of 4.5-6.5 is reached in the eyedrops which is prepared by mixing the pirenoxine-containing suspension and the solvent. Preferably, the pH is in the range of 5.6-6.4. In achieving this, the pH of the pirenoxine-containing suspension is usually at or lower than 5.5, preferably in the range of 2-4.5 for example, and more preferably in the range of 3-5. The pH of the solvent is preferably in the range of 5-8, and more preferably in the range of 5.5-6.5. Adjustment of the pH of each of the compositions to be mixed (the pirenoxine-containing suspension and the solvent in the case of the present invention) so that the pH of the mixed solution falls within an aimed range can be made with ease by, for example, distributing, as desired, a desired amount of the components (esp. buffers, pH adjusting agents, etc.) which serve to achieve the abovementioned proper pH of the mixed solution, in accordance with the pH set for the respective compositions to be mixed (for example, the buffer is included only in the solvent).

The pirenoxine-containing suspension and the solvent according to the present invention may contain additives generally employed in ophthalmic preparations, such as buffers, suspending agents, isotonic agents, stabilizers, preservatives, pH adjusting agents, etc.

Examples of buffers include phosphate buffer, citrate buffer, borate buffer, carbonate buffer, ε-aminocaproic acid, aminoethylsulfonic acid, glutamic acid, borax, and the like. Among these, phosphate buffer, citrate buffer, borate buffer and aminoethylsulfonic acid are preferred.

Examples of suspending agents include water soluble polymers such as hydroxypropylmethylcellulose, methylcellulose, hydroxyethylcellulose, hydroxypropylcellulose, propylcellulose, carboxymethylcellulose, polyvinylpyrrolidone, polyvinylalcohol, and the like;, surfactants such as polysorbate 80, tyloxapol, polyoxyethylenehydrogenated castor oil, polyethyleneglycol monostearate, sucrose fatty acid esters, and the like.

Examples of isotonic agents include sodium chloride, potassium chloride, glycerol, mannitol, sorbitol, propylene glycol, boric acid, glucose, and the like.

Examples of stabilizing agents include sodium edetate, sodium citrate, sodium sulfite, sodium bisulfite, sodium thiosulfate, ascorbic acid, and the like.

Examples of preservatives include methyl p-hydroxybenzoate, ethyl p-hydroxybenzoate, propyl p-hydroxybenzoate, chlorobutanol, benzalkonium chloride, benzethonium chloride, benzyl alcohol, sodium dehydroacetate, boric acid, borax, and the like.

Examples of pH adjusting agents include hydrochloric acid, acetic acid, citric acid, phosphoric acid, sodium hydroxide, potassium hydroxide, sodium carbonate, and the like.

Though the amount of the abovementioned additives to be added varies depending on their types and aims, they may be added in an amount in which they can achieve their aims. An isotonic agent is generally added to the pirenoxine-containing suspension or to the solvent or to both of them so that the relative osmotic pressure of from about 0.8 to about 1.2 is reached in the eyedrops prepared by mixing the pirenoxine-containing suspension and the solvent. A buffering agent is added in an amount of from about 0.01 to about 5 w/v% to the pirenoxine-containing suspension or to the solvent or to both of them. A suspending agent is added in an amount of from about 0.0001 to about 1 w/v% to the pirenoxine-containing suspension or to the solvent or to both of them. A stabilizer is added in an amount of from about 0.001 to about 1 w/v% to the pirenoxine-containing suspension, or to the solvent, or to both of them. A preservative is added in an amount of from 0.0005 to about 0.5 w/v% to the pirenoxine-containing suspension or to the solvent or to both of them.

Each of the pirenoxine-containing suspension and the solvent which forms the mixed-before-use-type, two-liquid ophthalmic preparation according to the present invention may be produced a method well known to those skilled in the art. For example, they may be produced as desired by the methods described in the section of Eyedrops, Suspensions, or Liquids and Solutions, in General Rules for Preparations, the Japanese Pharmacopoeia 15th Edition.

The mixed-before-use-type, two-liquid ophthalmic preparation consisting of a pirenoxine-containing suspension and a solvent according to the present invention may be provided in the form in which the pirenoxine-containing suspension and the solvent are separately contained in the respective compartments of a container which is provided with two compartments and allows to mix their contents before use. Examples of such a container includes a container described in, e.g., Japanese Patent Application Publication No. H11-292154, which is a type of container including integrally provided 2 or more compartments interconnected via a sealed portion which are so made to open a communication path under pressure from outside due to detachment of sealed layers. If the mixed-before-use-type, two-liquid ophthalmic preparation consisting of a pirenoxine-containing suspension and a solvent according to the present invention is provided in such a container, there is an advantage as it will prevents such accidents as contamination with foreign matters during mixing, leak of the eyedrops, and inadvertent skip of the mixing process. However , the mixed-before-use-type, two-liquid ophthalmic preparation according to the present invention may also be provided in the form in which the pirenoxine-containing suspension and the solvent are contained in separate containers.

The present invention will be described in further detail below with reference to Examples and Test Examples. It is not intended, however, that the present invention be limited to those examples.

### [Examples]

### Test Example 1. Solubility Test

### (Preparation of Ophthalmic Preparations for Testing)

According to the formulas shown in Table 1, each ophthalmic preparation was prepared.

### [TABLE 1]

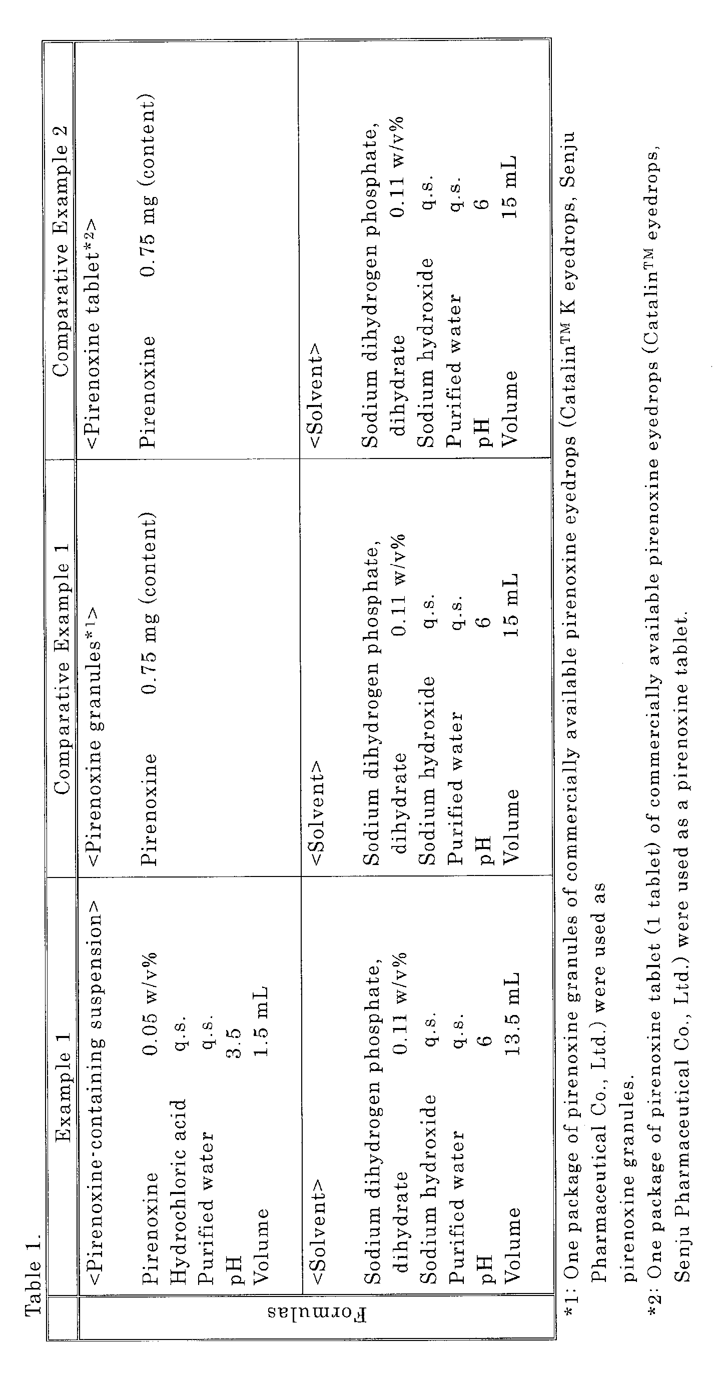

### (Test Method)

The pirenoxine-containing suspension or granules or tablet, and the solvent which constituted each ophthalmic preparation were mixed in a 20-mL beaker and stirred at 25 °C and at the stirring rate of 600 (VARIOMAG, POLY) with a cylindrical stirrer bar (ca. 2 cm in length × ca. 0.5 cm in diameter). The length of time required for pirenoxine to dissolve was measured in each mixture solution. In addition, pH of each eyedrops obtained 10 min after the start of mixing was measured. The concentration of pirenoxine in all the eyedrops thus formed after mixing was 0.005 w/v%.

The results are shown in Table 2.

### [TABLE 2]

**Table 2.**

| | Example 1 | Comparative Example 1 | Comparative Example 2 |
|---|---|---|---|
| Dissolution time | Within 5 seconds | 15 seconds | Not less than 60 seconds |
| pH after dissolution | 5.75 | 5.91 | 5.91 |

As shown in Table 2, in the case of the ophthalmic preparation of Example 1, pirenoxine dissolved within 5 seconds after the suspension and the solvent were mixed. The pH of the eyedrops after 10 min of mixing was 5.75.
On the other hand, in the case of the ophthalmic preparation of Comparative Example 1 (granules), the dissolution time of pirenoxine was 15 seconds. Furthermore, in the case of the ophthalmic preparation of Comparative Example 2 (tablet), it took not less than 60 seconds to dissolve pirenoxine.
These results indicate that a mixed-before-use-type, two-liquid ophthalmic preparation consisting of a pirenoxine-containing suspension and a solvent enables to reduce the time required to dissolve pirenoxine after mixing, as compared with those types of ophthalmic preparations consisting of a pirenoxine-containing tablet or pirenoxine-containing granules and a solvent.

### Test Example 2. Solubility Test

### (Preparation of Ophthalmic Preparations for Testing)

According to the formula shown in Tables 3, 4 and 5, mixed-before-use-type, two-liquid ophthalmic preparations were prepared.

### [TABLE 3]

**Table 3.**

| Formulas | | Example 2 | Example 3 | Example 4 |
|---|---|---|---|---|
| Suspension | Pirenoxine | 0.05 w/v% | 0.01 w/v% | 0.0056 w/v% |
| | Hydrochloric acid | q.s. | q.s. | q.s. |
| | Purified water | q.s. | q.s. | q.s. |
| | pH | 3.5 | 3.5 | 3.5 |
| | Volume | 1.5 mL | 7.5 mL | 13.5 mL |
| Solvent | Sodium dihydrogen phosphate, dihydrate | 0.11 w/v% | 0.2 w/v% | 1.0 w/v% |
| | Sodium hydroxide | q.s. | q.s. | q.s. |
| | Purified water | q.s. | q.s. | q.s. |
| | pH | 6 | 6 | 6 |
| | Volume | 13.5 mL | 7.5 mL | 1.5 mL |
| Mixing ratio | Suspension / Solvent (ratio in volume, v/v) | 1:9 | 5:5 | 9:1 |

### [TABLE 4]

**Table 4.**

| Formulas | | Example 5 | Example 6 | Example 7 |
|---|---|---|---|---|
| Suspension | Pirenoxine | 0.05 w/v% | 0.01 w/v% | 0.0056 w/v% |
| | Hydrochloric acid | q.s. | q.s. | q.s. |
| | Purified water | q.s. | q.s. | q.s. |
| | pH | 3.5 | 3.5 | 3.5 |
| | Volume | 1.5 mL | 7.5 mL | 13.5 mL |
| Solvent | Sodium citrate | 0.11 w/v% | 0.2 w/v% | 1.0 w/v% |
| | Sodium hydroxide | q.s. | q.s. | q.s. |
| | Purified water | q.s. | q.s. | q.s. |
| | pH | 6 | 6 | 6 |
| | Volume | 13.5 mL | 7.5 mL | 1.5 mL |
| Mixing ratio | Suspension / Solvent (ratio in volume, v/v) | 1:9 | 5:5 | 9:1 |

### [TABLE 5]

**Table 5.**

| Formulas | | Comparative Example 3 | Comparativ e Example 4 | Comparative Example 5 |
|---|---|---|---|---|
| Suspension | Pirenoxine | 0.05 w/v% | 0.01 w/v% | 0.0056 w/v% |
| | Hydrochloric acid | q.s. | q.s. | q.s. |
| | Purified water | q.s. | q.s. | q.s. |
| | pH | 3.5 | 3.5 | 3.5 |
| | Volume | 1.5 mL | 7.5 mL | 13.5 mL |
| Solvent | Sodium acetate | 0.11 w/v% | 0.2 w/v% | 1.0 w/v% |
| | Sodium hydroxide | q.s. | q.s. | q.s. |
| | Purified water | q.s. | q.s. | q.s. |
| | pH | 6 | 6 | 6 |
| | Volume | 13.5 mL | 7.5 mL | 1.5 mL |
| Mixing ratio | Suspension / Solvent (ratio in volume, v/v) | 1:9 | 5:5 | 9:1 |

The test method was the same as that in Test Example 1.

The results of the test are shown in Tables 6 and 7.

### [TABLE 6]

**Table 6.**

| | Example 2 | Example 3 | Example 4 | Example 5 | Example 6 | Example 7 |
|---|---|---|---|---|---|---|
| Dissolution time | Within 5 seconds | Within 5 seconds | Within 5 seconds | Within 5 seconds | Within 5 seconds | Within 5 seconds |
| pH after mixed | 5.75 | 5.67 | 5.89 | 5.93 | 5.90 | 6.17 |

### [TABLE 7]

**Table 7.**

| | Comparative Example 3 | Comparative Example 4 | Comparative Example 5 |
|---|---|---|---|
| Dissolution time | Not less than 60 seconds | Not less than 60 seconds | Not less than 60 seconds |
| pH after mixed | 5.82 | 5.72 | 5.68 |

As shown in Table 6, in any of the cases of the ophthalmic preparations of Examples 2-4, in which the solvent was phosphate buffer, and the ophthalmic preparations of Examples 5-7, in which the solvent was citrate buffer, pirenoxine dissolved within 5 seconds after the suspension and the solvent were mixed.
On the other hand, as shown in Table 7, in all the cases of the ophthalmic preparations of Comparative Examples 3-5, in which the solvent was acetate buffer, it took not less than 60 seconds to dissolve pirenoxine.

### Test Example 3. Solubility Test

### (Preparation of Ophthalmic Preparations for Testing)

According to the formulas shown in Table 8, mixed-before-use-type, two-liquid ophthalmic preparations were prepared.

### [TABLE 8]

**Table 8.**

| Formulas | | Example 8 | Example 9 |
|---|---|---|---|
| Suspension | Pirenoxine | 0.05 w/v% | 0.01 w/v% |
| | Hydrochloric acid | q.s. | q.s. |
| | Purified water | q.s. | q.s. |
| | pH | 3.5 | 3.5 |
| | Volume | 1.5 mL | 7.5 mL |
| Solvent | Boric acid | 1.43 w/v% | 2.56 w/v% |
| | Borax | 0.005 w/v% | 0.009 w/v% |
| | Potassium chloride | 0.224 w/v% | 0.4 w/v% |
| | Propyl p-hydroxybenzoate | 0.011 w/v% | 0.02 w/v% |
| | Methyl p-hydroxybenzoate | 0.022 w/v% | 0.04 w/v% |
| | Aminoethylsulfonic acid | 0.55 w/v% | 0.98 w/v% |
| | Sodium hydroxide | q.s. | q.s. |
| | Phosphoric acid | q.s. | q.s. |
| | Purified water | q.s. | q.s. |
| | pH | 6 | 6 |
| | Volume | 13.5 mL | 7.5 mL |
| Mixing ratio | Suspension / Solvent (ratio in volume, v/v) | 1:9 | 5:5 |

### (Test method)

The test method was the same as that in Example 1

The results of the test are shown in Table 9.

### [TABLE 9]

**Table 9.**

| | Example 8 | Example 9 |
|---|---|---|
| Dissolution time | Within 5 seconds | Within 5 seconds |
| pH after mixed | 5.97 | 6.35 |

As shown in Table 9, in either of the cases of the ophthalmic preparations of Examples 8 and 9, pirenoxine dissolved within 5 seconds after the suspension and the solvent were mixed.

### INDUSTRIAL APPLICABILITY

According to the present invention, a mixed-before-use-type, two-liquid ophthalmic preparation consisting of pirenoxine-containing suspension and a solvent enables to reduce the time required to dissolve pirenoxine, as compared with those types of conventional ophthalmic preparations with which a tablet or granules comprising pirenoxine are dissolved in a solvent before use. And, further, the present invention enables to provide a clear eyedrops with weakly acidic to practically neutral pH, which is physiologically acceptable, after dissolution of pirenoxine.

## Claims

1. A mixed-before-use-type, two-liquid ophthalmic preparation consisting of a pirenoxine-containing suspension and a solvent.

2. The ophthalmic preparation according to claim 1 which is adjusted so that the pH of the eyedrops prepared by mixing the pirenoxine-containing suspension and the solvent is 4.5-6.5.

3. The ophthalmic preparation according to claim 1 or 2, wherein the mixing ratio in volume of the pirenoxine-containing suspension to the solvent is 1:9 to 9:1 (v/v).

4. The ophthalmic preparation according to one of claims 1 to 3, wherein the concentration of pirenoxine in the pirenoxine-containing suspension is 0.005-0.05 w/v%.

5. The ophthalmic preparation according to one of claims 1 to 4, wherein the pH of the pirenoxine-containing suspension is 2-4.5.

6. The ophthalmic preparation according to one of claims 1 to 5, wherein the pH of the solvent is 5-8.

7. A mixed-before-use-type, two-liquid ophthalmic preparation which is an ophthalmic preparation consisting of a pirenoxine-containing suspension and a solvent, wherein the pirenoxine-containing suspension and the solvent are separately contained in different compartments of a container which is provided with two or more compartments which can be made into communication with one another to mix their contents before use
